# EUROPEAN PATENT APPLICATION

(11) **EP 0 648 766 A1**
(43) Date of publication of application: **19.04.1995**
(21) Application number: 94913621.2
(22) Date of filing: 26.04.1994
(51) Int. Cl.: C07D 405/12, A61K 31/495

(54) **AGENT ACTING ON THE CENTRAL NERVOUS SYSTEM, METHOD FOR ITS PREPARATION AND PHARMACEUTICAL COMPOSITIONS CONTAINING SUCH AGENT**

(30) Priority: 29.04.1993 ES 9300906
(71) Applicant: VITA-INVEST, S.A., E-08970 Sant-Joan-Despi (ES)
(72) Inventor: CALDERO GES, José, Maria, E-08034 Barcelona (ES); MONGE VEGA, Antonio, E-31190 Cizur-Menor (ES); DEL RIO ZAMBRANA, Joaquin, E-28036 Madrid (ES); PALOP CUBILLO, Juan, Antonio, E-31007 Pamplona (ES); LASHERAS ALDAZ, Berta, E-31020 Baranain (ES); ARTAIZ URDACI, Inés, E-31003 Pamplona (ES); RAMIREZ MEDRANO, Carmen, E-01008 Vitoria (ES); ROCA ACIN, Juan, E-08006 Barcelona (ES); BOSCH ROVIRA, Ana, E-08017 Barcelona (ES)
(74) Representative: Ponti Sales, Adelaida
(86) International application number: ES9400040
(87) International publication number: WO9425454

(57) **Abstract**

A new 2-piperazinilindol derivative, the 3-[2-(1-(4'-piperonilpiperazinil))indolyl]carboxaldehyde, having the formula (I) and the pharmaceutically acceptable salts thereof, the method for its preparation, its utilization as an anxiolytic, antidepressant and memory stimulating agent, and the compositions containing it.

## Description

This invention refers to a new derivative of 2-piperazinylindol, 3-[2-(1-(4'-piperonylpiperazinyl)) indolyl]-carboxaldehyde, useful as an agent acting on the central nervous system.

In accordance with the invention, a compound of formula (I) is provided, 3-[2-(1-(4'-piperonylpiperazinyl)) indolyl]-carboxaldehyde: 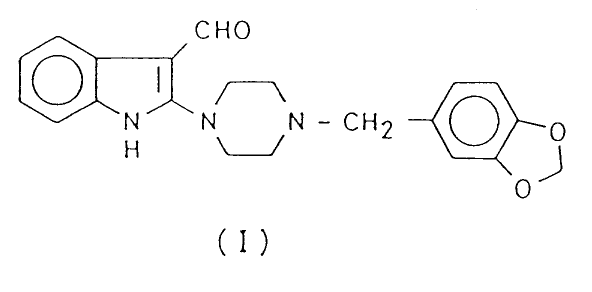
and its pharmaceutically acceptable salts.

### BACKGROUND OF THE INVENTION

Patent ES 454234, applied for on 14.12.76 (Hoechst), describes and claims a method to obtain an indole derivative of general formula (II): 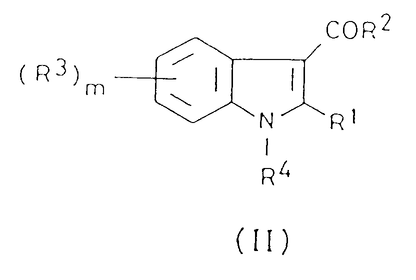
where R¹, R², R³, R⁴ and m represent a multitude of possible replacements, due to which this general formula takes in millions of different products, of which only a very small number are specifically described.

It is stated in said patent ES 454234 that these products derived from Indole possess valuable pharmacological properties, being especially efficacious for cardiac circulation.

The product object of the present patent, 3-[2-(1-(4'-piperonylpiperazinyl))indolyl]-carboxaldehyde, is not described in the aforesaid patent ES 454234, although it is included within the scope of the general formula claimed in that patent.

### DESCRIPTION OF THE INVENTION

It has been found that the 3-[2-(1-(4'-piperonylpiperazinyl))indolyl]-carboxaldehyde, object of the present invention, possesses suprisingly interesting pharmacological properties, especially in the treatment of anxiety disorders, depression and as a memory stimulant.

This invention also provides a pharmaceutical composition including a compound of formula (I) or a pharmaceutically acceptable salt of same together with a pharmaceutically acceptable diluent. The composition is preferably presented in the form of tablets, capsules, injectable liquid, solution or suspension.

The invention of formula (I) also provides a pharmaceutical compound of formula (I) or a pharmaceutically acceptable salt of same for use in the treatment of anxiety disorders, depression and as a memory stimulant.

The compound of formula (I) can be obtained by two different procedures. In a general process (A), in accordance with this invention, it is obtained by nucleophilic replacement reaction of a leaving group X in position 2, such as chlorine, bromine, methanosulphonate or toluenesulphonate, of the indolic compound of formula (III) with piperonyl-piperazine (IV).

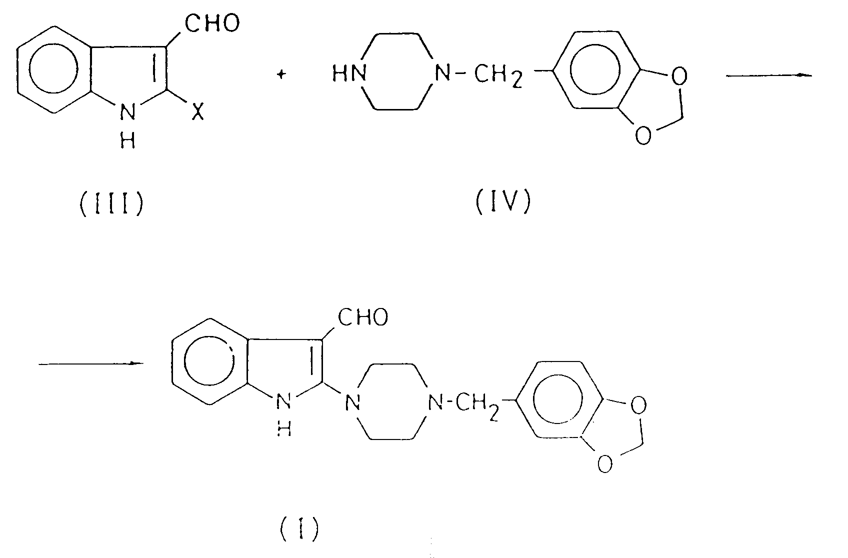

The starting compound (III) is obtained from 2-oxoindole (V), by treatment with phosphorus oxychloride and N,N-dimethylformamide or N,N-dimethylacetamide in a chloroform medium, as can be found described in the literature (G.M. Coppolo and G.S. Hardimann, J. Heterocyclic. Chem., 14. 1117 (1977)).

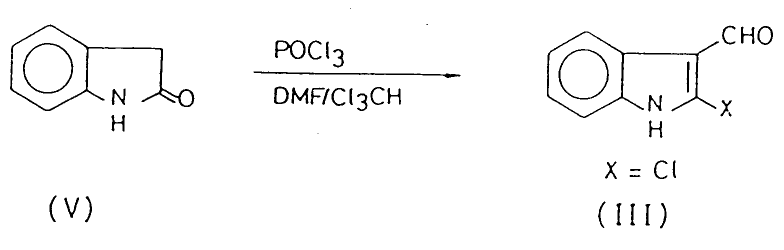

In accordance with another general process (8), the compound (I) can also be obtained by alkylation of the piperazinylindole (VI) (obtained by nucleophilic replacement of the chlorine of compound (III) with piperazine) with an alkylating agent such as 4-chloromethyl-1,2-(methylendioxy)benzene (VII):

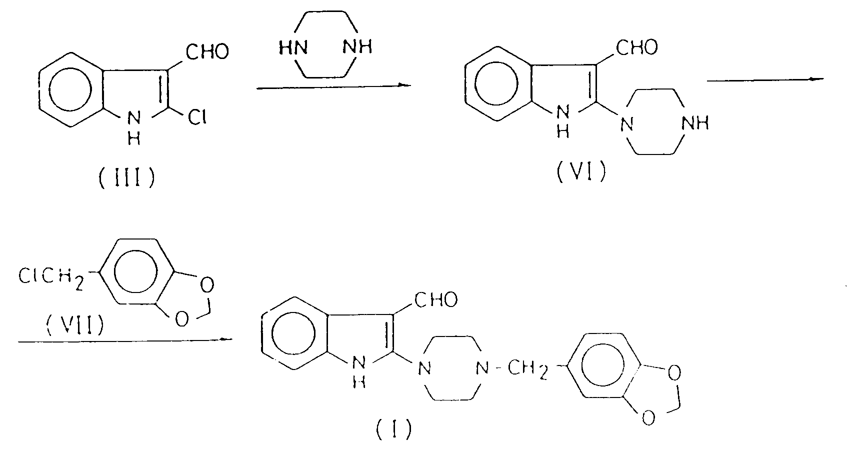

The preferred pharmaceutically acceptable salts are the acid addition salts. The pharmaceutically acceptable addition salts of the compounds of formula (I) are those formed from acids which form non-toxic addition salts containing pharmaceutically acceptable anions. The salts may derive from inorganic acids such as hydrochloric, hydrobromic, sulphuric or nitric acid, or from organic acids such as lactic, succinic, oxalic, maleic acid, etc.

The salts may be obtained by the conventional methods, as for example by mixing solutions containing equimolar quantities of the free base and the desired acid. The salt formed is separated by filtration, if it is insoluble, or by evaporation of the solvent.

The compound of formula (I) and its pharmaceutically acceptable salts are highly active as anxiolytics, antidepressants and memory stimulators.

### EXPERIMENTAL PART

The following preparation is described by way of example in order to illustrate the invention.

### Example 1

A mixture of 1 g (5.5 mm) of 2-chloroindolyl-3-carboxaldehyde and 2.42 g (0.011 mm) of N-piperonyl-piperazine is heated to melting point. The mixture is left to cool and 10 ml of ethanol is added. The mixture is left to stand for 24 hours. It is treated with 10 ml of a 0.1N solution of caustic soda, and the mixture is stirred at room temperature for two hours. The product thus obtained is separated by filtration to give 0.8 g (40% yield) of 3-[2-(1-(4'-piperonylpiperazinyl))indolyl]-carboxaldehyde in the form of an orange solid. Melting point 222-3°C (recrystallization from ethanol/N,N-dimethylformamide).
- ¹H-RMN: (DMSO-d₆, 200 MHz) δ (ppm): 11.301 (s, 1H, N-H); 9.958 (S, 1H, CHO); 7.956 - 7.944 (d, 1H, indole); 7.231 - 6.787 (m, 6H, indole + 1,3,4-thisubstituted benzene ring); 6.003 (s, 2H, methylendioxide); 3.590 (bd. s., 4H piperazin); 3.436 (s, 2H, methylene); 2.527 (bd. s., 4H, piperazine).
- IR: 3123 (m, N-H); 2932-2803 (m, aliphatic CH); 1595 (s, C=O); 1568-1387 (s, aromatic quart.); 1237-1039 (m, C-O); 863-809 (w, 1,3,4-trisubstitution)cm⁻¹.

| Element analysis: C₂₁H₂₁N₃O₃ | | | |
|---|---|---|---|
| % Calculated: | C: 69.421, | H: 5.785 | N: 11.570 |
| % Found: | C: 69.437, | H: 5.927 | N: 11.506 |

### Pharmacological results

### 1) Anxiolytic activity. Two-compartment box.

The method used is that of Crawley, J.N.; Pharmacol. Biochem. Behav., 15, 695-699 (1981). This consists of a two-compartment box with different lighting, counting the time of stay and number of entries into the light and dark compartments, the number of lifting movements and the number of lines crossed in each of the two compartments, before and after treatment with the products under study. The percentage of time spent in the light compartment compared to that spent by the same group before the treatment; while in the control group that time diminishes significantly in the 2nd exposure, this does not occur with an anxiolytic treatment.

| PRODUCT | DOSE AND VIA | N | % TIME IN LIGHT COMPARTMENT (X ± E.S.) |
|---|---|---|---|
| Excipient | PRE-EXPOSURE | 10 | 21,6 ± 4,2 |
| | Excipient i.p. | | 5,7 ± 1,4 |
| ONDANSETRON | PRE-EXPOSURE | 10 | 16,6 ± 3,3 |
| | 2µg/kg i.p. | | 12,3 ± 2,7 |
| TROPISETRON | PRE-EXPOSURE | 20 | 12,4 ± 3,0 |
| | 10 µg/kg i.p. | | 12,3 ± 3,0 |
| COMPOUND I | PRE-EXPOSURE | 20 | 15,0 ± 2,0 |
| | 100 µg/kg i.p. | | 8,6 ± 3,2 |
| | PRE-EXPOSURE | 20 | 22,9 ± 3,1 |
| | 50 µg/kg i.p. | | 15,4 ± 4,4 |
| | PRE-EXPOSURE | 11 | 10,7 ± 3,5 |
| | 20 µg/kg i.p. | | 12,7 ± 6,5 |
| | PRE-EXPOSURE | 11 | 10,8 ± 3,6 |
| | 2 µg/kg i.p. | | 14,6 ± 7,2 |
| ONDANSETRON | PRE-EXPOSURE | 10 | 21,1 ± 8,0 |
| | 4 µg/kg v.o. | | 18,7 ± 5,1 |
| COMPOUND I | PRE-EXPOSURE | 11 | 24,9 ± 6,8 |
| | 8 µg/kg v.o. | | 28,6 ± 8,0 |
| | PRE-EXPOSURE | 10 | 14,5 ± 4,0 |
| | 4 µg/kg v.o. | | 20,0 ± 5,5 |

### 2) Anxiolytic activity. Social interaction test.

Use is made of the method of File, S.E. and colls.; J. Neurosci. Methods. 2, 219-238 (1980). This consists in placing a pair of rats in an unknown and strongly lit environment. Automatic recording is carried out of social activity under these conditions of strong lighting compared with the control animals.

| PRODUCT | DOSE AND VIA | N | SOCIAL INTERACTION TIME (sec ± E.S.) |
|---|---|---|---|
| Excipient | Excipient i.p | 10 | 158 ± 15 |
| DIAZEPAM | 2 mg/kg i.p. | 6 | 257 ± 22 |
| BUSPIRONA | 0,25 mg/kg i.p. | 6 | 287 ± 19 |
| | 1 mg/kg i.p. | 6 | 275 ± 14 |
| ONDANSETRON | 0,01 mg/kg i.p. | 6 | 247 ± 22 |
| | 0,05 mg/kg i.p. | 5 | 219 ± 18 |
| TROPISETRON | 0,01 mg/kg i.p. | 6 | 253 ± 10 |
| | 0,1 mg/kg i.p. | 5 | 231 ± 26 |
| COMPOUND I | 0,5 mg/kg i.p. | 8 | 235 ± 12 |
| | 2 mg/kg v.o. | 6 | 262 ± 17 |

### 3) Antidepressive activity. Desperate behaviour induced in mouse by enforced swimming. Porsolt test.

Use is made of the method of Porsolt, R.D. and colls.; Nature, 266, 730-732 (1977). This consists in subjecting the mice to a swimming test by placing them inside transparent cylinders of set dimensions, with water at a temperature of 20-22°C. After a prior detection test, the time the animal takes to adopt an immobile posture is determined, recording the duration of immobility. The average immobility time of the treated groups is compared with that of the control group.

| PRODUCT | DOSE AND VIA | N | IMMOBILITY TIME (sec ± E.S.) |
|---|---|---|---|
| Excipient IMIPRAMINA COMPOUND I | - i.p. | 12 | 132,53 ± 8,70 |
| | 30 mg/kg i.p. | 11 | 71,72 ± 9,82 |
| | 10 mg/kg i.p. | 10 | 136,60 ± 11,26 |
| | 1 mg/kg i.p. | 10 | 59,80 ± 11,00 |
| Excipient COMPOUND I | - i.p. | 6 | 89,70 ± 21,7 |
| | 0,5 mg/kg i.p. | 10 | 31,60 ± 10,2 |
| | 0,1 mg/kg i.p. | 10 | 49,4 ± 17,8 |

### 4) Effect on learning and memory. Passive avoidance test on rats.

Use is made of the method of Chugh and colls., Eur. J. Pharmacol., 203, 121-123 (1991). This consists in placing the rats in a two-compartment (light and dark) box separated by a door which opens and closes as the experimenter wishes; the floor of both cages is constructed using a metal grid electrifiable as desired in terms of current and duration. The animal is conditioned to avoid the dark zone, and the time of latency in the exposures following the one in which the animals receive the electric shock of 0.2 mA and duration of 3 seconds is determined.

| PRODUCT | DOSE AND VIA | N | DATA TYPE | 1st LATENCY 3rd LATENCY -(24 h) | 2nd LATENCY (10') |
|---|---|---|---|---|---|
| Excipient | - i.p. | 7 | average ± E.S. median | 7,1 ± 1,6 | 5,7 ± 1,5 |
| | | | | 10,7 ± 4,8 | |
| | | | | 9 | 6 |
| | | | | 21 | |
| GRANIS ETRON | 0,1 mg/kg i.p. | 6 | average ± E.S. median | 6,3 ± 1,1 | 8,5 ± 1,8 |
| | | | | 19,0 ± 6,5 | |
| | | | | 8 | 11 |
| | | | | 21 | |
| COMPOUND I | 1 mg/kg i.p. | 7 | average ± E.S. median | 2,7 ± 0,7 | 3,3 ± 1,4 |
| | | | | 23,6 ± 9,0 | |
| | | | | 3 | 4 |
| | | | | 42 | |

### 5) Acute toxicity

The acute toxicity is determined by different vias in mice.

| PRODUCT | VIA | APPROXIMATE DL₅₀ (mg/kg) |
|---|---|---|
| ONDANSETRON | i.p. | 7,5 |
| GRANISETRON | i.p. | 89 |
| ZACOPRIDE | i.p. | 135 |
| COMPOUND I | i.p. | > 500 |
| COMPOUND I | v.o. | > 500 |

## Claims

1. The 3-[2-(1-(4'-piperonylpiperazinyl))indolyl]-carboxaldehyde, of formula (I). 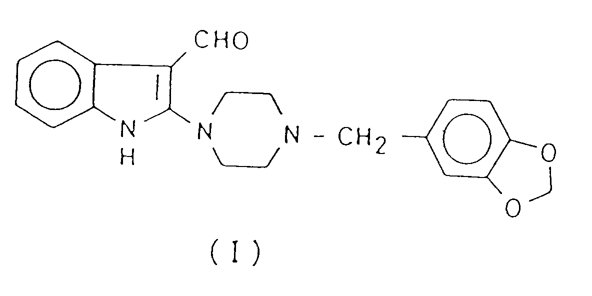 and its pharmaceutically acceptable salts.

2. A method for preparation of the compound of formula (I), as claimed in Claim 1, comprising the reaction of an indolic compound of formula (III) with piperonylpiperazine (IV). 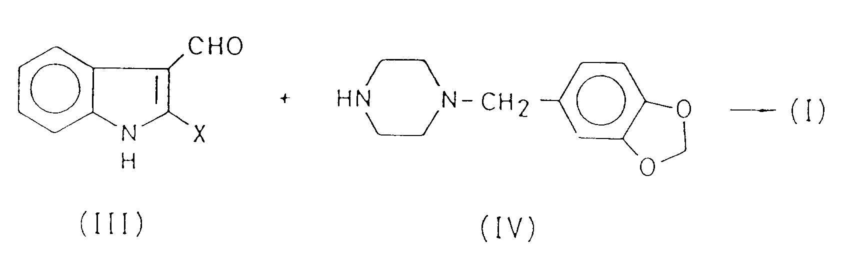 in which X is a leaving group, such as chlorine, bromine, methanesulphonate or p-toluenesulphonate.

3. A method for the preparation of formula (I), as claimed in Claim I, which comprises the reaction of an indolic compound of formula (IV) with 4-halomethyl-1,2(methylendioxy) benzene (VII). 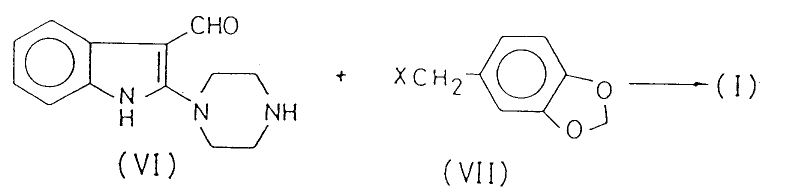 where X is a halogen, such as fluorine, chlorine, bromine or iodine.

4. A pharmaceutical composition which includes the compound of formula (I), as claimed in Claim 1, or one of its pharmaceutically acceptable salts, together with a pharmaceutically acceptable diluent.

5. A composition, as claimed in Claim 4, which is beneficial for human use preferably in the form of tablets, capsules, injectable liquid, solution or suspension.

6. The use of a compound of formula (II), as claimed in Claim 1, or one of its pharmaceutically acceptable salts, as anxiolytic, antidepressive and memory stimulator.
